# EUROPÄISCHE PATENTSCHRIFT

(11) **EP 1 304 073 B1**
(45) Veröffentlichungstag und Bekanntmachung des Hinweises auf die Patenterteilung: **29.03.2006**
(21) Anmeldenummer: 02023416.7
(22) Anmeldetag: 18.10.2002
(51) Int. Cl.: A61B 5/0482, A61B 5/00

(54) **Biofeedback-Verfahren und Vorrichtung, sowie Verfahren zur Erzeugung und Darstellung von Daten**
Biofeedback method and device, as well as a method for producing and presenting data
Procédé et dispositif de rétroaction biologique, ainsi que procédé pour produire et présenter des données

(30) Priorität: 19.10.2001 DE 10151152
(43) Veröffentlichungstag der Anmeldung: 23.04.2003
(73) Patentinhaber: Slawinski, Andrezj, Dr., 7056 Molinis (CH)
(72) Erfinder: Slawinski, Andrzej Dr., 7056 Molinis (CH); Klöckner, Wolfgang, 78464 Konstanz (DE)
(74) Vertreter: Muri, Peter

(56) Entgegenhaltungen:
- WO-A-01/08417
- WO-A-02/15985
- US-A- 5 702 323
- US-A- 5 899 867
- US-A- 6 011 991

## Beschreibung

Die Erfindung bezieht sich auf eine Vorrichtung zur Anwendung eines Biofeedback-Verfahrens gemäß dem Oberbegriff von Anspruch 1 sowie einem Verfahren zur Verwendung der Vorrichtung des Anspruchs 1 gemäß dem Oberbegriff des Anspruchs 14.

### Definition von Grundbegriffen

Für die nachfolgende Beschreibung, sowie Ansprüche und Zeichnungen werden Grundbegriffe verwendet, die zur Ktarstellung nachfolgend näher erläutert werden:

### Biofeedback-Verfahren:

Biofeedback ist ein wissenschaftlich fundiertes Verfahren mit dessen Hilfe in der Regel unbewußt ablaufende psychophysiologische Prozesse durch Rückmeldung (Feedback) wahrnehmbar gemacht werden. Nach dem Prinzip der operanden Konditionierung können diese Vorgänge dann gezielt beeinflußt und ihre Regulation bis zur willentlichen Kontrolle erlernt werden. Insbesondere findet das psychoregulative Verfahren im Sport Anwendung. Der große Vorteil des Biofeedback-Verfahrens ist, dass mehrere psychophysiologische Parameter genau erfasst und in Sekundenbruchteilen rückgemeldet werden können. Das Beeinflussen der einzelnen Parameter benötigt eine unterschiedliche Anzahl von Trainlngseinheiten, wobei man auch individuelle Unterschiede zwischen Personen und auf Unterschiede bei den Parametern nehmen muß. Die einzelnen Verfahren müssen genau auf die Personen abgestimmt werden und weisen die größten Erfolge auf, wenn unter Aufsicht entsprechend trainiert wird. Insbesondere günstige Anwendungsmöglichkeiten bieten diejenigen Sportarten, bei denen die Konzentrationsfähigkeit einen wichtigen Faktor darstellt und die nur kleine Bewegungsumfänge beanspruchen.

Ziel der Anwendung von Biofeedback und anderen psychoregulativen Verfahren ist es, eine psychische Stabilität des Sportlers bei Wettkämpfen zu erreichen. Der Sportler soll in der Lage sein, sich in einen Aktivierungszustand zu versetzen und somit sein Potential voll auszuschöpfen. Befindet sich der Sportler in einem für die Leistungen günstigen Zustand, soll er leistungshindemde Faktoren erkennen und verändem können. Die Befähigung dafür kann durch Biofeedback und andere psychoregulative Verfahren gelernt werden. Die Anwendung des Biofeedback-Verfahrens findet auch Einzug bei der Persönlichkeitsgestaltung. Nicht nur im Bereich des Sports sind entsprechende Konzentrationsfähigkeiten notwendig, um Leistung zu erbringen. Daher kann das Biofeedback-Verfahren auch als psychoregulatives Verfahren zur Leistungsoptimierung von jeglichen Personen eingesetzt werden, die beispielsweise im Arbeitsleben leistungsorientiert tätig sind.

### Psychophysiologische Parameter:

Meßbare, körpereigene Signale, die mit psychischen / mentalen Zuständen oder Aktivitäten zusammenhängen. Diese Signale werden mit numerischen Methoden analysiert und parametrisiert (z.B. Puls zu Herzrate. EEG zu Frequenzbändern, Hautwiderstand zu lokalen Änderung etc.)

### Psychointeraktivität:

Eine Erweiterung des Begriffs der Interaktivität auf die psychischen und mentalen Prozesse. Die Psychointeraktivität kann zwischen Mensch und Mensch, aber auch zwischen Mensch und Computer oder anderen Mehrbenutzem/Computer Netzwerkkombination stattfinden.

### Sensoren:

Die Sensoren dienen dazu, die Signale (psychophysiologische Parameter) zu erfassen und sie in elektrische Signale umzuwandeln.

### Softwareeinheit:

Die Softwareeinheit besteht aus einer Software und einer Rechnereinheit/ einem Computer, die die Software lauffähig gestaltet. In dieser Softwareeinheit werden die von den Sensoren übermittelten elektrischen Signale mittels den, in der Software implementierten Algorithmen analysiert, umgerechnet und als bearbeitete Daten, sogenannte Aktivdaten bereitgestellt.

### Funktionsmodul:

Das Funktionsmodul dient dazu, Aktivdaten, die von der Softwareeinheit bereitgestellt werden, zusammenzufassen, eventuell auszuwerten und an die Darstellungseinheit der jeweiligen anderen Benutzer weiterzureichen.- Abhängig von der jeweiligen Einstellung ist es möglich, dass die Aktivdaten derart ausgewertet werden, dass diese zu einem Gruppendatenwert zusammengefasst werden und diese auf die Darstellungseinheit eines jeweiligen Benutzers, der der Gruppe zugeordnet ist, projiziert werden. Auf diese Weise können die Benutzer einer Gruppe das gruppendynamische Verhalten als Gruppe betrachten. Alternativ hierzu ist vorgesehen, die Aktivdaten der einzelnen Benutzer, wahlweise einer Gruppe zugehörig oder beliebig, auf der Darstellungseinheit der arideren Benutzer darzustetlen. Auf diese Weise können die Benutzer das Verhalten anderer Benutzer wahrnehmen und gegebenenfalls analysieren. Das Funktionsmodul besteht ebenfalls aus einer Software und einer Rechnereinheit und verfügt über entsprechende Speicherkapazität, sowie die notwendigen Anschlüsse.

### Stand der Technik

Zur Zeit befinden sich auf dem Markt einige Biofeedback-Systeme meistens zu Therapie-, For-schungs-, oder Trainingszwecken eingesetzt.

Die verwendeten Methoden dienen dazu, psychophysiologischen Parametern zu erfassen, beispielsweise bei Puls, Atem, Temperatur, Hautwiderstand oder ähnliches. In der Regel ist vorgesehen, dass beispielsweise der Hautwiderstand eines einzelnen Benutzers gemessen wird, die entsprechenden Signale über Sensoren erfasst werden und über eine Softvvareeinheit entsprechende Aktivdaten bereitgestellt werden, die beispielsweise auf einem Monitor darstellbar sind. Durch die Wahrnehmung des Benutzers mittels seiner Sinnesorgane ist es möglich, dass der Benutzer Einfluss nimmt auf seine psychophysiologischen Parameter und durch die interaktiven Prozesse zwischen der Darstellung der Aktivdaten auf der Därstellungseinheit bzw. dem Monitor seiner Wahrnehmung und der Abgabe seiner psychophysiologischen Parameter den interaktiven Prozess steuern können.

Erste Anwendungen des Biofeedback-Verfahrens finden bereits bei Spielen, sogenannte Mental Games, Neurogames Einzug. In der Regel wird jedoch das Biofeedback - Verfahren von einer einzigen Person zu Trainings-, Therapie- oder Forschungszwecken verwendet.

Aus der WO 02/15985 A1 ist ein System und ein Verfahren bekannt, das dazu dient, mittels Trainingsgeräten, wie beispielsweise einem Lauftrainer gewonnene Ergebnisse mit anderen Trainingspartnem, die auf den gleichartigen Geräten trainieren, zu vergleichen. Hierfür ist vorgesehen, dass die einzelnen Sportgeräte mit Anzeigeeinrichtungen ausgerüstet und über ein Netzwerk beispielsweise schnurlos (wireless) miteinander verbunden sind und zwar In der Weise, dass die einzelnen Daten auf einer gemeinsamen Rechnereinheit abgelegt werden und dort über eine Anzeigeeinrichtung darstellbar sind. Ferner besteht die Möglichkeit, dass die so übertragenen und dargestellten Daten von einer weiteren Person, beispielsweise einem Trainer während des Trainings oder auch danach analysiert werden können. Es ist auch die Möglichkeit vorgesehen, dass dieser Trainer eine Rückmeldung über den Trainingszustand auf die Anzeigeeinrichtung des Trainingsgeräts geben kann. Insbesondere ist dieses System dafür vorgesehen. Trainingsgeräte ausserhalb eines Fitnessstudios zu verwenden und trotzdem unter der Beobachtung eines qualifizierten Trainers zu stehen.

Aus der WO 01/08417 A1 ist ein System sowie ein Verfahren bekannt, das dazu dient, Sportler während ihrer sportlichen Tätigkeit zu überwachen. Hierzu ist vorgesehen, dass über jeden Sportler physiologische Daten gesammelt werden (beispielsweise durch eine Sensoreinheit, wobei die Daten telemetrisch an eine zentraale Einheit übermittelt werden), diese anschliessend übermittelt und ausgewertet werden. Ferner ist vorgesehen. Videoaufnahmen mit den ausgewarteten Daten in Übereinstimmung zu bringen. Dieses System dient dazu, über visuell (beispielsweise Videobilder und Herzfrequenz) bereitgestellte Daten die Belastung eines Sportlers festzustellen.

Aus der US 5,702,323 entnimmt der Fachmann die Lehre, eine Einrichtung zu schaffen, mittels der die physikalischen Daten eines Sportlers schnurlos an eine Recheneinheit übertragen werden können und dort mit weiteren Parametern verglichen werden können. Das Ergebnis kann der Sportler während seiner Übung auf einer Anzeigeeinheit einsehen.

In der US 6,011,911 ist eine Vorrichtung dargestellt, mittels der Benutzer untereinander kommunizieren können, wobei als Kommunikationsmittel Gehimaktivitäten oder -wellen eingesetzt werden.

Aus der US 5,899,876 ist ein System bekannt, das dafür vorgesehen ist, dass die Gehimaktivitäten eines Benutzers auf einem Monitor darstellt, aufzeichnet und entsprechend ausewrtet.

In US 5,443,076 ist eine Biofeedbackvorrichtung sowie ein Verfahren offenbart, welches sich auf die Aufzeichnung der elektrischen Tätigkeit des Gehirns bezieht.

### Nachteile des Stands der Technik

Gemäß dem Stand der Technik sind bisher Biofeedback-Technologien nur zur Steuerung von Softwareeinherten nur für Einzelpersonen bekannt.

Ein wesentlicher Nachteil besteht darin, dass die Aktivdaten ausschließlich zur Steuerung einfacher Parameter, wie beispielsweise eines Cursors auf einem Monitor, einer Tonleiter oder Steuerung einer Kugel mit unterschiedlichen Geschwindigkeiten oder ähnliches verwendet werden.

Eine Einbeziehung von mehreren Benutzern zur Steuerung eines gemeinsamen Parameters bzw. von gemeinsamen Parametern ist nicht bekannt.

### Aufgabe der Erfindung

Daher besteht die Aufgabe der Erfindung darin, das Biofeedback-Verfahren gemäß dem Stand der Technik derart weiterzuentwickeln, dass dieses für Trainingszwecke einsetzbar ist.

### Lösung der Aufgabe

Die Aufgabe der Erfindung wird durch den kennzeichnenden Teil des Anspruchs 1 sowie durch den kennzeichnenden Teil des Anspruchs,14 gelöst.

### Vorteile der Erfindung

Durch die Anwendung der sogenannten Psychointeraktivität der Benutzer, die zu einem Netzwerk zusammengeschlossen sind, ist es jetzt möglich mit ganzen Gruppen und im Gegensatz zum Stand der Technik nicht nur mit einzelnen Benutzern zu arbeiten und hier das Biofeedback-System auch gruppenweise zu nutzen. Dies bringt den Vorteil mit sich, dass nicht nur die von einem einzigen Benutzer bereitgestellten psychophysiologischen Parameter ausgewertet werden, sondern auch durch die Zusammenfassung der in einer Gruppe mittels eines Gruppenmoduls zusammengefaßten Benutzer die einzelnen psychophysiologischen Parameter erfasst werden, zu einem einzigen Aktivdatenparameter, der vom Gruppenverhalten abhängt, zusammengefaßt werden und so, interaktiv, das Gruppenverhalten der einzelnen Benutzer untersucht und trainiert werden kann.

Alternativ hierzu ist auch vorgesehen, die ermittelten Aktivdaten der an dem Biofeedback-System konnektierten Benutzer auf die Darstellungseinheiten der anderen Benutzer zu übertragen, so dass der jeweilige Benutzer im Stande ist, sich ein Bild über die an dem System beteiligten Personen zu bilden.

Diese erfindungsgemäße Erneuerung lässt zu, dass auch mentale und psychische Vorgänge der Benutzer dazu verwendet werden können, komplexe Programme, insbesondere Multimediaanwendungen zu steuern. Die Programmabläufe könnten auch dazu verwendet werden, dass Gruppen miteinander oder gegeneinander trainieren, insbesondere über psychointeraktive Spiele, das entsprechende Gruppenverhalten realisiert und trainiert werden kann.

Eine vorteilhafte Weiterbildung besteht darin, dass die Ober das Funktionsmodul gewonnenen Gruppenaktivdaten nicht nur in Form von Parametern, beispielsweise auch Monitoren darstellbar sind, sondern auch in Form von Multimediaanwendungen, beispielsweise über Ton. Zusätzlich kann vorgesehen sein, dass die zu einer Gruppe zusammengeschlossenen Benutzer sich unterhalten und beeinflussen können oder aber dass andere Gruppen oder Einzelpersonen über Sprache (beispielsweise über Ton oder Schrift) beeinflußbar sind. Diese Beeinflussung kann auch von einem Mentor, der extem, d.h. nicht den Gruppen zugeordnet ist, vorgenommen werden.

Es ist daher Ziel der Einrichtung, die Gruppendynamik der einzelnen Benutzer zentral oder dezentral zu erfassen und darzustellen. Diese Art der Ausführung kann bei Seminaren Anwendung finden, wobei es hierzu nicht notwendig ist, dass die Benutzer in einem Raum physikalisch vorhanden sind, sondern auch virtuell. Hierzu ist vorgesehen, beispielsweise über interaktives Fernsehen die Benutzer zu koppeln.

Auf Grund der Komplexität des erfindungsgemäßen Biofeedback-Systems ist auch möglich, dass die Benutzer die sich zu entsprechenden Gruppen zusammenschließen, nicht unmittelbar an einem Ort vorhanden sein müssen mittels Intranet- bzw. Internetlösung aber auch über Datenfernübertragungen ist es möglich, dass sich unterschiedliche Benutzer von unterschiedlichen Orten zusammenschließen und hier entsprechende Trainings-, Forschungs- und sonstige Maßnahmen mit Hilfe des Biofeedback-Verfahrens durchführen.

Zusätzlich Können unterschiedliche Simulationstechniken auf den Darstellungseinheiten vorgesehen sein, die die mentalen Zustände der einzelnen Benutzer bzw. Gruppen fordert bzw. hindert. Dadurch wird erreicht, dass ein bestimmtes Brain-Storming, Problemanalyse oder Kreativitäten geweckt werden. Damit können die Benutzer in sogenannte Simmulationszustände versetzt werden.

Eine wesentliche Weiterentwicklung des erfindungsgemäßen Feedback-Systems besteht darin, dass auch psychointeraktive "Femsehprogramme" realisierbar sind, in dem sich das Programm automatisch dem Zuschauer bzw. dem Benutzer anpasst, bzw. indem es beispielsweise dessen Gemütszustand erfasst und dann eine entsprechende Kategorie von Programmen auswählt und zur Verfügung stellt.

Eine Erweiterung der erfindungsgemäßen Vorrichtung zur Anwendung des Biofeedback-Verfahrens kann auch darin bestehen, dass die psychophysiologischen Parameter mit neuen oder für die Biofeedback-Methode noch untypischen Methoden gewonnen werden könne, zum Beispiel MEG, ultraschwache Photolumeniszenz, auch ohne Elektroden oder Implantate, oder mit Femsensoren oder durch entsprechende biometrische Erkennungssysteme. Voraussetzung hierfür ist, dass diese Systeme Informationen über psycho-mentale Zustände des Benutzers tiefem und diese dann in entsprechende Daten umwandelbar sind, so dass sie adaptiv an die erfindungsgemäße Biofeedback-Vorrichtung bzw. Mithilfe dessen erfindungsgemäßen Verfahrens angeschlossen werden können.

Das erfindungsgemäße Biofeedback-System bzw. das hierauf basierende Verfahren lässt sich auch in den Bereichen der Schulen, des Sports, von Seminaren und von anderen Formen der Ausbildung oder Trainings anwenden, um so neue Möglichkeiten im Umgang mit Konzentration, Entspannung, Anregung und anderen psycho-mentalen Zustände, die zum Lernen vorteilhaft sind kennenzulemen, zu trainieren und nach schon relativ kurzer Zeit gezielt anwenden zu können.

Weitere Vorteilhaft Ausgestaltungen gehen aus der nachfolgenden Beschreibung, sowie den Ansprüchen und der Zeichnung hervor.

### Zeichnung

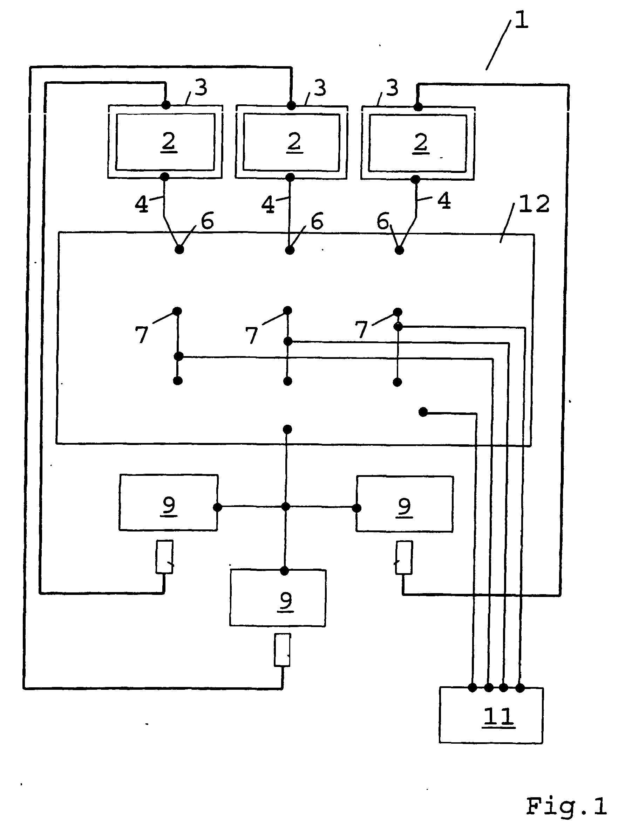

Es zeigt
- **Fig. 1**: eine Prinzipdarstellung der erfindungsgemäßen Vorrichtung zur Anwendung eines Biofeedback-Verfahrens, insbesondere für mehrere Benutzer.

### Beschreibung eines Ausführungsbeispiels

In der Figur ist eine Vorrichtung 1 zur Anwendung eines Biofeedback-Verfahrens dargestellt. Diese Vorrichtung 1 umfasst einzelne Sensoreinheiten 2, die jeweils von Benutzern 3 psychophysiologische Parameter erfassen und dies Ober elektrische Leitungen 4 oder wahlweise auch schnurlos per Datenübertragung an eine Softwareeinheit weiterleiten. Diese Softwareeinheit umfasst eine Schnittstelle 6, die zur Aufnahme der über die Datenleitung 4 übermittelten psychophysiologischen Parameter vorgesehen ist, sowie eine weitere Schnittstelle 7 zur Abgabe und Weiterleitung von Aktivdaten, die auf der Grundlage der psychophysiologischen Parameter mittels einer in der Softwareeinheit integrierten Software errechnet worden sind. Für die Errechnung dieser Parameter kann unter anderem sogenannte Fast-Fourier-Transformationen (FFT) eingesetzt werden, um beispielsweise eine Spektralanalyse der hier vorgegebenen psychophysiologischen Parameter vorzunehmen.

Es ist vorgesehen die Softwareeinheit entweder einem Benutzer 3 unmittelbar zuzuordnen oder aber die Softwareeinheit als Serverkonfiguration auszubilden, so dass die Parameter, die von den Sensoren 2 über die Datenleitung 4 ermittelt werden zentral in der Softwareeinheit erfasst und bearbeitet werden können.

Die von der Softwareeinheit nun über die Schnittstelle 7 bereitgestellten Aktivdaten werden an ein Funktionsmodul weitergeleitet. Dieses Funktionsmodul ist derart ausgelegt, dass mittels dieser Einrichtung Benutzer 3 in einzelne Gruppen/ Teams zusammengefaßt werden können und aus den Aktivdaten sogenannte Gruppenaktivdaten ermittelt werden, die dann an den jeweiligen Darstellungseinheiten 9, die wiederum den einzelnen Benutzern 3 zugeordnet sind, aufgezeigt werden. Alternativ hierzu ist abhängig von der jeweiligen Einstellung des Funktionsmoduls auch vorgesehen, die Aktivdaten der anderen Benutzer unbearbeitet weiterzuleiten, so dass sich jeder Benutzer über die weiteren Benutzer insbesondere über dessen psychophysiologischen Parameter ein Bild machen kann.

Auf diese Art und Weise lässt sich auf sehr einfache Art und Weise eine entsprechende Team- oder Gruppenbildung generieren, so dass die psychophysiologischen Parameter von den einzelnen Teammitgliedem zusammengefaßt werden und nur das Gesamtverhalten des Teams bzw. der Gruppe als Ganzes in Form von Gruppenaktivdaten entsprechend dargestellt werden. Eine Teambildung ist auch ohne Ermittlung und Errechnung von Gruppenaktivdaten möglich, in dem die Aktivdaten der jeweils einer Gruppe zugehörigen Benutzer auf den Darstellungseinheiten der jeweiligen Gruppen aktiv angezeigt werden. Auf diese Art ist der Benutzer im Stande, entweder das gruppendynamische Verhalten oder aber das Verhalten eines jeden der Gruppe zugehörigen Benutzers einzuschätzen.

Die Darstellung erfolgt auf Darstellungseinheiten 9, beispielsweise in Form von Multimediaanwendungen, in Form von Bild, Ton, oder sonstigen Reizen. Da diese Reize unmittelbar auf den Benutzer beispielsweise über seinen Sinnesapparat (Augen -> Sehen) wirken, ist eine entsprechende Interaktivität zwischen der Darstellungseinheit 9, dem Benutzer 3 bzw. den Sensoren 2 gegeben.

Zusätzlich kann ein Mental Koordinator 11 vorgesehen sein, der mittels beeinflussenden Maßnahmen die Interaktivität der Benutzer beeinflussend eingreifen indem er beispielsweise auf der Darstellungseinheit entsprechende Vorgänge ändert, durch Meta-Sprache die Sinnesorgane der einzelnen Benutzer 3 beeinflußt oder entsprechende sonstige Reize ausübt.

Erfindungsgemäß ist vorgesehen, dass die Softwareeinheit und das Funktionsmodul eine zentrale Mentaleinheit 12 bilden, die als Serverlösung konfiguriert ist und beispielsweise einem Internetserver entspricht. Dies bedeutet, dass jeder Benutzer 3 mit einem hier in der Zeichnung nicht näher dargestellten Interface sich in den Mentalserver 12 einloggen kann und hier mit einfacher Menüsteuerung ein Training durchführen kann, wobei wahlweise es ihm möglich ist, allein das Biofeedback-Verfahren durchzuführen oder aber in einem Team, bzw. in einer Gruppe oder aber mit einem Mental Koordinator 11.

Die Internetlösung lässt sich auch auf eine Intranetlösung ohne weiteres übertragen. An Stelle des Intranets bzw. des internets können auch Datenfemübertragungseinrichtungen vorgesehen sein, die sich dadurch auszeichnen, dass ein Remote-Access-Server installiert ist, der mittels drahtloser Konnektierung oder beispielsweise einer Telefonleitung für jeden Benutzer 3 erreichbar ist.

Dass die Vorrichtung zur Anwendung eines Biofeedback-Verfahrens für mehrere Benutzer sowie das Verfahren zur Erzeugung und Darstellung von Daten auf eine Darstellungseinheit bei der Anwendung eines Biofeedback-Verfahrens für mehrere Benutzer zeichnet sich durch einen hohen Grad von Flexibilität aus. Zudem ergeben sich hieraus Möglichkeiten, die durch die bisherige Art von Feedback-Verfahren, wie sie aus dem Stand der Technik bekannt sind, nicht möglich sind.

## Patentansprüche

1. Vorrichtung zur Anwendung eines Biofeedback Verfahrens, bestehend im wesentlichen aus
- mindestens eines Sensors zur Erfassung von psychophysiologischen Parametern des jeweiligen Benutzers und Weiterteilung als elektrische Signale,
- einer Softwareeinheit zur Erfassung dieser elektrischen Signale und Verarbeitung der elektrischen Signale und Weiterleitung der bearbeiteten Signale als Aktivdaten und
- einer Darstellungseinheit zur Darstellung der Aktivdaten in Form von Bild, Ton oder sonstigen Reizen zur Herbeiführung einer interaktiven Verbindung zwischen den mit der Darstellungseinheit aufgezeigten Daten, den Sinnesorganen des Benutzers und den Sensoren, und
- einem Funktionsmodul (12) das die von der Softwareeinheit (12) bereitgestellten aktivdaten von einer definierten Anzahl von mehreren Benutzern (3) zusammenführt,
**dadurch gekennzeichnet, dass**
- die dargestellten Aktivdaten von einer Herzrate oder von einer lokalen Änderung des Hautwider stands abhängig sind, einer
- durch das Funktionsmodul (12) Gruppen von Benutzern bildbar sind und die so zusammengeführten Aktivdaten zu einen Gruppendatenwert ausbildbar sind, und
- das Functionsmodul (8) diesen Gruppendatenwert an die die Gruppe bildenden Benutzer (3) auf deren Darstellungseinheit (9) weiterleitet.

2. Vorrichtung nach Anspruch 1, **dadurch gekennzeichnet, dass** die Softwareeinheit (12) ein Berechnungsverfahren umfasst, mittels dem die psychophysiologischen Parameter in Aktivdaten umgewandelt werden.

3. Vorrichtung nach Anspruch 3, **dadurch gekennzeichnet, dass** das Berechnungsverfahren ein Fast-Fourier-Transformationsverfahren ist.

4. Vorrichtung nach mindestens einem der vorhergehenden Ansprüche, **dadurch gekennzeichnet, dass** die Aktivdaten, die von der Softwareeinheit (12) errechnet aus den psychophysiologischen Parametern errechnet werden, zur Steuerung von Multimediaanwendungen vorgesehen ist

5. Vorrichtung nach mindestens einem der vorhergehenden Ansprüche, **dadurch gekennzeichnet, dass** eine Meta-Sprache zur Kommunikation unter den Benutzern (3) vorgesehen ist.

6. Vorrichtung nach mindestens einem der vorhergehenden Ansprüche, **dadurch gekennzeichnet, dass** die Softwareeinhelt (12) und das Funktionsmodul (12) eine Zentrale Mentaleinheit (12) bilden.

7. Vorrichtung nach Anspruch 6, **dadurch gekennzeichnet, dass** die Mentaieinheit (12) als Serverkunfiguration ausgebildet ist.

8. Vorrichtung nach Anspruch 7, **dadurch gekennzeichnet, dass** die Mentaleinheit (12) ein Internet/Webserver ist.

9. Vorrichtung nach mindestens einem der vorhergehenden Ansprüche, **dadurch gekennzeichnet dass** die Datenübertragung der psychophysiotogischen Parameter, sowie die Gruppendatenwerte über das internet erfolgt

10. Vorrichtung nach mindestens einem der vorhergehenden Ansprüche, **dadurch gekennzeichnet, dass** die Datenübertragung der psychophysiologischen Parameter, sowie die Gruppendatenwerte über das Intranet erfolgt

11. Vorrichtung nach mindestens einem der vorhergehenden Ansprüche, **dadurch gekennzeichnet, dass** die Datenübertragung der psychophysiologischen Parameter, sowie die Gruppendatenwerte über Datenfernübertragung erfolgt

12. Vorrichtung nach mindestens einem der vorhergehenden Ansprüche, **dadurch gekennzeichnet, dass** die Datenübertragung der psychophysiologischen Parameter, sowie die Gruppendatenwerte schnurlos/drahtlos erfolgt.

13. Vorrichtung nach mindestens einem der vorhergehenden Ansprüche, **dadurch gekennzeichnet, dass** ein Mental Koordinator (11) vorgesehen ist, der die psychophysiologischen Parameter der einzelnen Benutzer durch Manipulation der Gruppendatenwerte ändert.

14. Verfahren zur Verwendung der Vorrichtung des Anspruchs 1, und nicht zur therapeutischen Behandlung des menschlichen oder tierischen Körpers, zur Erzeugung und Darstellung von Daten auf einer Darstellungseinheit bei der Anwendung eines Biofeedback-Verfahrens mit mehreren Benutzern, **gekennzeichnet durch folgende Verfahrensschritte:**
a Erfassen von psychophysiologischen Parametern mittels jeweils einer den Benutzern (3) angeordneten Sensoreinheit (2), und Weiterleitung der psychophysiologischen Parameter als elektrische Signale,
b Erfassen dieser elektrischen Signale mittels einer Softwareeinheit (12) und Errechnen von Aktivdaten mittels des von den Sensoreinheiten (2) als elektrische Signale bereitgestellten psychophysiologiologischen parametern mittels einer softwareeinheit (5) und Übergabe der Aktivdaten an ein Funktionsmodul (12),
c Zusammenfassen dieser Ackivadaten zu Gruppenaktivdaten mittels Funklionsmodul (12) wobei das Funktionsmodul (12) dievon der Softwareeinheit (12) bereitgesteiiten Aktivdaten von iner definlerten Anzahl von Benutzern (3) zusammenführt und hieraus die Gruppenaktivdaten bildet,
d Darstellen der Aktivdaten oder der Gruppenaktivdaten auf jeweils einer, der jeweiligen Benutzer und/oder die Gruppe bildenden Darstellungseinhalten (9).

## Claims

1. Apparatus for applying a biofeedback method, essentially comprising
- at least one sensor for recording psychophysiological parameters from the respective user and forwarding them as electrical signals,
- a software unit for recording these electrical signals and processing the electrical signals and forwarding the processed signals as active data, and
- a presentation unit for presenting the active data in the form of pictures, sound or other stimuli for bringing about an interactive link between the data displayed using the presentation unit, the user's sensory organs and the sensors, and
- a functional module (12) which brings together the active data provided by the software unit (12) from a defined number of a plurality of users (3),
**characterized in that**
- the presented active data are dependent on a heart rate or on a local change in the resistance of the skin, the functional module (12) can form groups of users and can develop the active data brought together in this way to form a group data value, and
- the functional module (12) forwards this group data value to the users (3) forming the group on their presentation unit (9).

2. Apparatus according to Claim 1, **characterized in that** the software unit (12) comprises a computation method which is used to convert the psychophysiological parameters into active data.

3. Apparatus according to Claim 2, **characterized in that** the computation method is a Fast Fourier Transformation method.

4. Apparatus according to at least one of the preceding claims, **characterized in that** the active data which are calculated from the psychophysiological parameters by the software unit (12) are provided for controlling multimedia applications.

5. Apparatus according to at least one of the preceding claims, **characterized in that** a meta-language is provided for communication among the users (3).

6. Apparatus according to at least one of the preceding claims, **characterized in that** the software unit (12) and the functional module (12) form a central mental unit (12).

7. Apparatus according to Claim 6, **characterized in that** the mental unit (12) is in the form of a server configuration.

8. Apparatus according to Claim 7, **characterized in that** the mental unit (12) is an Internet/web server.

9. Apparatus according to at least one of the preceding claims, **characterized in that** the data transmission for the psychophysiological parameters, and also the group data values, is effected via the Internet.

10. Apparatus according to at least one of the preceding claims, **characterized in that** the data transmission for the psychophysiological parameters, and also the group data values, is effected via the intranet.

11. Apparatus according to at least one of the preceding claims, **characterized in that** the data transmission for the psychophysiological parameters, and also the group data values, is effected using remote data transmission.

12. Apparatus according to at least one of the preceding claims, **characterized in that** the data transmission for the psychophysiological parameters, and also the group data values, is effected cordlessly/wirelessly.

13. Apparatus according to at least one of the preceding claims, **characterized in that** a mental coordinator (11) is provided which changes the psychophysiological parameters of the individual users by manipulating the group data values.

14. Method for using the apparatus from Claim 1, and not for the therapeutic treatment of the human or animal body, for generating and presenting data on a presentation unit when applying a biofeedback method with a plurality of users, **characterized by** the following method steps:
a psychophysiological parameters are recorded using a respective sensor unit (2) arranged for the users (3), and the psychophysiological parameters are forwarded as electrical signals,
b these electrical signals are recorded using a software unit (12), and active data are calculated by means of a software unit (12) using the psychophysiological parameters provided by the sensor units (2) as electrical signals, and the active data are transferred to a functional module (12),
c these active data are combined to form group active data by means of the functional module (12), with the functional module (12) bringing together the active data provided by the software unit (12) from a defined number of users (3) and forming the group active data therefrom,
d the active data or the group active data are presented on one of the respective presentation units (9) forming users and/or the group.

## Revendications

1. Dispositif pour l'application d'un procédé de rétroaction biologique essentiellement constitué
- d'au moins un capteur pour l'enregistrement de paramètres psychophysiologiques de l'utilisateur du moment et acheminement sous forme de signaux électriques,
- d'une unité logicielle pour l'enregistrement de ces signaux électriques et pour le traitement des signaux électriques et pour l'acheminement des signaux traités sous forme de données actives, et
- d'une unité d'affichage pour l'affichage des données actives sous forme d'image, de son ou d'autre stimuli pour provoquer une liaison interactive entre les données présentées par l'unité d'affichage, les organes sensoriels de l'utilisateur et les capteurs, et
- d'un module fonctionnel (12) qui regroupe les données actives d'un nombre défini de plusieurs utilisateurs (3), préparées par l'unité logicielle (12)
**caractérisé en ce que**
- les données actives affichées dépendent d'un rythme cardiaque ou d'une modification locale de la résistance de la peau, et
- à l'aide du module fonctionnel (12), on peut constituer des groupes d'utilisateurs et on peut développer les données actives ainsi regroupées sous la forme d'une valeur des données du groupe, et
- le module fonctionnel (12) achemine cette valeur de données du groupe aux utilisateurs (3) constituant le groupe sur leur unité d'affichage (9).

2. Dispositif selon la revendication 1, **caractérisé en ce que** l'unité logicielle (12) englobe une méthode de calcul permettant de convertir les paramètres psychophysiologiques en données actives.

3. Dispositif selon la revendication 2, **caractérisé en ce que** la méthode de calcul est une méthode de transformation de Fourier rapide.

4. Dispositif selon une au moins des revendications précédentes, **caractérisé en ce que** les données actives calculées par l'unité logicielle (12) à partir des paramètres psychophysiologiques sont prévues pour piloter des applications multimédia.

5. Dispositif selon une au moins des revendications précédentes, **caractérisé en ce qu'**un méta-langage est prévu pour la communication entre les utilisateurs (3).

6. Dispositif selon une au moins des revendications précédentes, **caractérisé en ce que** l'unité logicielle (12) et le module fonctionnel (12) constituent une unité mentale centrale (12).

7. Dispositif selon la revendication 6, **caractérisé en ce que** l'unité mentale (12) est formée en configuration serveur.

8. Dispositif selon la revendication 7, **caractérisé en ce que** l'unité mentale (12) est un serveur web/internet.

9. Dispositif selon une au moins des revendications précédentes, **caractérisé en ce que** la transmission des données des paramètres psychophysiologiques ainsi que les valeurs des données des groupes est assurée par Internet.

10. Dispositif selon une au moins des revendications précédentes, **caractérisé en ce que** la transmission des données des paramètres psychophysiologiques ainsi que les valeurs des données des groupes est assurée par Intranet.

11. Dispositif selon une au moins des revendications précédentes, **caractérisé en ce que** la transmission des données des paramètres psychophysiologiques ainsi que les valeurs des données des groupes est assurée par transmission de données.

12. Dispositif selon une au moins des revendications précédentes, **caractérisé en ce que** la transmission des données des paramètres psychophysiologiques ainsi que les valeurs des données des groupes est assurée sans fil.

13. Dispositif selon une au moins des revendications précédentes, **caractérisé en ce qu'**un coordinateur mental (11) est prévu qui modifie les paramètres psychophysiologiques des utilisateurs individuels par manipulation des valeurs des données des groupes.

14. Procédé pour l'application du dispositif de la revendication 1, et non pas pour le traitement thérapeutique du corps humain ou animal, pour la production et pour l'affichage de données sur une unité d'affichage en utilisant un procédé de rétroaction biologique avec plusieurs utilisateurs, **caractérisé par** les étapes suivantes du procédé :
a. enregistrement de paramètres psychophysiologiques au moyen d'une unité de capteurs (2) disposée sur les utilisateurs (3) et acheminement des paramètres psychophysiologiques sous forme de signaux électriques,
b. enregistrement de ces signaux électriques au moyen d'une unité logicielle (12) et calcul par une unité logicielle (12) de données actives au moyen des paramètres psychophysiologiques préparés par les unités de capteurs (2) sous la forme de signaux électriques et transmission des données actives vers un module fonctionnel (12).
c. regroupement de ces données actives en données actives de groupes au moyen du module fonctionnel (12), le module fonctionnel (12) regroupant les données actives d'un nombre défini d'utilisateurs (3), préparées par l'unité logicielle (12), et en forme les données actives des groupes.
d. affichage des données actives ou des données actives des groupes sur respectivement une des unités d'affichage (9) des utilisateurs du moment et/ou des utilisateurs constituant les groupes.
